# EUROPEAN PATENT SPECIFICATION

(11) **EP 4 239 566 B1**
(45) Date of publication and mention of the grant of the patent: **17.12.2025**
(21) Application number: 22159399.9
(22) Date of filing: 01.03.2022
(51) Int. Cl.: G06T 5/50, G06T 5/73, G06T 5/92

(54) **COMPUTER-IMPLEMENTED METHOD FOR DETERMINING NUCLEAR MEDICAL IMAGE DATA SETS, IMAGING DEVICE, COMPUTER PROGRAM AND ELECTRONICALLY READABLE STORAGE MEDIUM**
COMPUTERIMPLEMENTIERTES VERFAHREN ZUM BESTIMMEN NUKLEARMEDIZINISCHER BILDDATENSÄTZE, BILDGEBUNGSVORRICHTUNG, COMPUTERPROGAMM UND ELEKTRONISCH LESBARES SPEICHERMEDIUM
PROCÉDÉ MIS EN UVRE PAR ORDINATEUR PERMETTANT DE DÉTERMINER DES ENSEMBLES DE DONNÉES D'IMAGES MÉDICALES NUCLÉAIRES, DISPOSITIF D'IMAGERIE, PROGRAMME INFORMATIQUE ET SUPPORT D'INFORMATIONS LISIBLE ÉLECTRONIQUEMENT

(43) Date of publication of application: 06.09.2023
(73) Proprietor: Siemens Healthineers AG, 91301 Forchheim (DE)
(72) Inventor: Bendriem, Bernard, Knoxville, TN, 37919 (US); Fenchel, Matthias, 91054 Erlangen (DE); Wehrl, Hans, 91052 Erlangen (DE)
(74) Representative: Siemens Healthineers Patent Attorneys

(56) References cited:
- HUNTER CHAD R R N ET AL: "Patient motion effects on the quantification of regional myocardial blood flow with dynamic PET imaging", MEDICAL PHYSICS, AIP, MELVILLE, NY, US, vol. 43, no. 4, 22 March 2016 (2016-03-22), pages 1829 - 1840, XP012206337, ISSN: 0094-2405, [retrieved on 19010101], DOI: 10.1118/1.4943565
- MANWELL SPENCER ET AL: "Whole-body motion correction in cardiac PET/CT using Positron Emission Tracking: A phantom validation study", 2018 IEEE NUCLEAR SCIENCE SYMPOSIUM AND MEDICAL IMAGING CONFERENCE PROCEEDINGS (NSS/MIC), IEEE, 10 November 2018 (2018-11-10), pages 1 - 3, XP033613130, DOI: 10.1109/NSSMIC.2018.8824715

## Description

The invention concerns a computer-implemented method for determining at least one nuclear medical image data set in nuclear medical imaging using an imaging device. The invention further concerns an imaging device, a computer program and an electronically readable storage medium.

Nuclear medical imaging, which may also be called emission tomography, is an imaging technique in which radio-actively labeled substances, so-called tracers, inside the body of a patient are detected. Examples for nuclear medical imaging comprise positron emission tomography (PET) and single photon emission computer tomography (SPECT). From the resulting nuclear medical raw data sets, for example sinogram data and/or list-mode data, which describe radiation measuring events, nuclear medical image data sets showing the distribution of the tracer can be reconstructed. In basic reconstruction approaches, backprojection, in particular filtered backprojection (FBP), may be employed. However, such images are often noisy, so that advanced, iterative reconstruction methods have been proposed, in particular MLEM (maximum likelihood expectation maximization) and/or OSEM (ordered subsets expectation maximization).

In some nuclear medical imaging applications, nuclear medical raw data of the same anatomical region are acquired in different acquisition steps, in particular separated in time.

For example, in dynamic nuclear medical imaging, a time period of progression of a tracer inside the patient may be divided into multiple frames as acquisition steps, wherein a nuclear medical image data set for each frame can be reconstructed from the nuclear medical raw data acquired in this frame. For reasons of clearer distinction between states of tracer distribution, a time interval may be provided between the acquisition times of different frames.

Furthermore, combined-modality workflows, for example PET-magnetic resonance (MR) workflows, have been proposed, in which nuclear medical raw data from an anatomical region is acquired in two different acquisition steps separated in time, in particular if regions of interest, which are larger than the field of view of the imaging device, are examined. In this case, multiple predefined positions of the field of view, such that the whole region of interest is covered, may be used for acquisition, in particular multiple predefined patient table positions. In particular, whole-body scans may be executed using imaging devices providing an additional imaging modality additional to the nuclear medical imaging modality, in particular PET-MR devices.

Here, it has been proposed to change the nuclear medical raw data assigned to different patient table positions with respect to the data volume in such a way that the recording times corresponding to the change to data volumes are matched to each other at different table positions, as described, for example, in US 8 781 195 B2. It was proposed to increase the recorded nuclear medical raw data assigned to a table position by further acquisition at this table position in another acquisition step.

In those cases, in other words, each anatomical region is imaged several times during an examination. However, a reading physician may want to obtain a single PET image with all summed counts of the individual passes for diagnostic purposes. Since the acquisition steps result in acquisition at different time points, changes may occur, in particular patient motion between different measurements of nuclear medical raw data sets of different acquisition steps. Furthermore, attenuation maps for attenuation correction are usually acquired at the beginning of the examination, in particular in a first acquisition step, and may not properly represent the motion state of each acquisition step. This could lead to nuclear medical image quality problems, for example blurring effects and/or quantification biases, for example in a final summed full-count image.

While, in the state of the art, it has already been proposed to provide motion correction regarding periodical physiological motions like respiration and/or cardiac motion within an acquisition step, for example using techniques like "Body-Compass", "OncoFreeze" or "CardiacFreeze", these approaches cannot account for image quality problems between acquisition steps.

Chad Hunter et al., "Patient motion effects on the quantification of regional myocardial blood flow with dynamic PET imaging", Medical Physics, AIP, Melville, NY, US, vol. 43, no. 4 , pages 1829 - 1840, ISSN: 0094-2405, DOI: 10.1118/1.4943565 discloses myocardial blood flow quantification from PET scans with CT attenuation correction including filtered back-projection and motion correction. Spencer Manwell et al., "Whole-body motion correction in cardiac PET/ CT using Positron Emission Tracking: A phantom validation study", Conference Proceedings, 2018 IEEE Nuclear Science Symposium and Medical Imaging (NSS/MIC), IEEE, 10 November 2018, pages 1 - 3, DOI: 10.1109/ NSSMIC.2018.8824715 discloses whole-body motion correction and image alignment from a PET/CT scanner using a radio-active tracer.

It is an object of the current invention to provide a method for increasing the image quality and spatial correspondence of nuclear medical image data sets based on raw data acquired in different acquisition periods, in particular spaced in time.

This object is achieved by providing a computer-implemented method, an imaging device, a computer program and an electronically readable storage medium according to the independent claims. Preferred embodiments are described by the dependent claims.

The invention concerns a computer-implemented method according to claim 1.

The at least one nuclear medical image data set relates to a certain motion state. It is proposed to use motion information from different acquisition steps to provide nuclear medical image data sets of improved quality and/or spatial correspondence. There is a time interval between the acquisition of the same sub-region of the region of interest, for example a certain position of the field of view with respect to the patient. While the method described here is also applicable to dynamic nuclear medical imaging, where the region of interest may be equal to or fully comprised by the field of view, due to the field of view being smaller than the region of interest, a certain time interval passes between acquisitions of the same sub-region of the region of interest, for example defined by a certain patient table position.

Hence, the region of interest is larger than the field of view of the imaging device, wherein, during each acquisition step, the field of view sweeps the whole region of interest by using multiple predefined positions of the field of view and/or continuous movement of the field of view with respect to the patient and/or wherein a series of nuclear medical image data sets is determined for all acquisition steps. Dynamic nuclear medical imaging can alsc be implemented for cases in which the field of view is smaller than the region of interest by repeatedly sweeping through the region of interest during the multiple acquisition steps, hence measuring nuclear medical raw data from different distribution states over the progression of the tracer in the region of interest. It is noted that these multiple passes of each sub-region can also be triggered or required by workflows as described in US 8 781 195 B2, but may advantageously be dedicatedly planned to allow dynamic nuclear medical imaging. Having, in particular complete, nuclear medical raw data sets for each acquisition step allows to reconstruct nuclear medical image data sets for each of these acquisition steps, such that a series of nuclear medical image data sets results, describing the evolution of the tracer in a large region of the body. Generally and preferably, the region of interest may comprise the whole body of a patient (whole-body examination head to thigh or full body head to toe). To determine a full-count image data set, the single nuclear medical image sets of the acquisition steps may be summed up. Summation may happen both in image and in raw data space. In this manner, a full-count nuclear medical image data set as well as information about the change of tracer distribution in time is provided.

According to the invention, the result is of a particular high quality despite possible motion in the region of interest, since motion data is determined and used for motion correction, such that nuclear medical image data sets from different acquisition steps can readily be compared and/or reconstructions can be combined.

It should be noted at this point that, preferably, the acquisition times for all acquisition steps are the same for the whole region of interest; however, in some cases, different acquisition times may be necessary or advantageous, such that, to enable comparison, further image processing and/or selection of nuclear medical raw data may be necessary.

As already mentioned, different predefined positions of the field of view defining sub-regions of the region of interest are selected by using different predefined patient table positions of a patient table on which the patient is placed for examination in the imaging device. For example, nuclear medical raw data of a nuclear medical raw data set of an acquisition step may be acquired for 0.5 to 2 minutes at each of, for example, two to seven predefined positions of the field of view, in particular the patient table. It has also been proposed to acquire nuclear medical imaging raw data over the whole region of interest by continuously moving the field of view, in particular the patient table, where the invention can, of course, also be employed.

Generally, the motion data may comprise vector fields for elastic motion and/or rigid body transformations for rigid body motion, in particular depending on anatomical features in the field of view. For example, rigid body transformations may be employed for the head of a patient. Combinations of vector fields for elastic motion and rigid body transformations are also conceivable, in particular for different anatomical features. For example, in the head area, rigid body transformations may be determined assuming the head as being a rigid body, while in the breast and/or abdomen area, elastic motion may be assumed such that vector fields best describe this elastic motion.

In preferred embodiments, the source data relating to one of the acquisition steps may be registered to source data from another acquisition step to determine motion data between the acquisition steps and/or optical flow algorithms may be used to determine the motion data. Hence, generally known motion estimation and/or detecting algorithms can also be used in the current invention, for example image-based registration algorithms and/or optical flow algorithms. As registration and/or optical flow are preferably evaluated in source data of the same modality, resource-saving implementations of the motion estimation and/or detection algorithms can be employed.

It should be noted already at this point that it is possible to use the nuclear medical imaging raw data, in particular images derived therefrom, as source data and/or to use source data from other modalities which are acquired anyways during the acquisition. The imaging device is a combined PET-MR device, magnetic resonance imaging data (MRI data) are acquired in parallel to the nuclear medical raw data sets. Hence, in such a case the current invention describes a straightforward extension of any multi-pass workflow in order to improve image quality and/or evaluability. Since the required source data is available anyways, the method can work without additional acquisitions or scans.

Preferably, if nuclear medical imaging data is to be used as source data, preliminary reconstructed images from the nuclear medical raw data sets, in particular backprojected images or other images determined by fast preliminary reconstruction, may be used as source data to register. Such preliminary reconstructed images, which are not yet motion-corrected, may hence serve as a basis for registering. Here, the image quality of simply backprojected images, for example by using filtered backprojection, or other fast preliminary reconstructed images already suffices to provide the necessary degree of precision in the motion data. Hence, readily available preliminary reconstructed images may be used without further effort and/or scans.

If a registration algorithm is used for motion estimation and/or detection, in preferred embodiments, neighboring acquisition steps may be registered in succession over the acquisition interval, in particular starting from the first or last acquisition step. If acquisition steps adjacent in time are registered, the least difference in motion state is expected, increasing robustness and reliability of the determination of the motion data. For example, source data from the first acquisition step may first be registered with source data from the second acquisition step, whereafter source data from the second acquisition step is registered to source data of the third acquisition step, and so on.

For applying the motion correction, multiple approaches are contemplated, wherein application of motion correction to the nuclear medical raw data sets and/or during reconstruction is preferred. It is, however, also conceivable to apply motion correction to an, in particular preliminary, reconstruction result in the image space. For example, image transformations may be applied before summation and/or comparison to reconstruction results of acquisition steps.

In preferred embodiments, motion correction is applied in raw data space during iterative reconstruction, in particular during motion-sensitive OSEM reconstruction and/or by warping an intermediate image result according to the motion data before forward projection and unwarping of correction terms after backprojection according to the motion data. Iterative reconstruction approaches are well-known in the state of the art and most often used in expectation maximization (EM) approaches, wherein an intermediate guess for the result image is forward projected to compare the nuclear medical raw data to the forward projected intermediate image data. Depending on this comparison, correction terms are applied to yield a new intermediate image as a guess for the real tracer distribution. Hence, if motion correction is to be applied during reconstruction while still being able to compare with the acquired nuclear medical raw data, motion correction is applied as described by warping the intermediate image result according to the motion data and then respectively unwarping correction terms. Motion-sensitive iterative reconstruction approaches have already been proposed, for example as motion-sensitive ordered subset expectation maximization (OSEM).

In other preferred embodiments, the nuclear medical raw data sets may be PET raw data sets, wherein, for motion correction, the lines of response are displaced according to the motion data. While the motion correction can, as described with respect to motion-sensitive OSEM above, be applied in sinogram space, it is also possible to apply motion correction in listmode space by reordering the lines of response of the events according to the motion data.

As already discussed, motion-corrected nuclear medical image data sets may be determined for multiple acquisition steps, in particular all acquisition steps. The motion data may be used to provide voxel-wise correspondence between nuclear medical image data sets for different acquisition steps. In this case, a series of dynamic nuclear medical images results, which are comparable and can be evaluated together. Alternatively or preferably, additionally, a motion-corrected nuclear medical image data set for multiple acquisition steps, in particular all acquisition steps, may be determined as a multi-count image data set, in particular a full count image data set. This can, as discussed, preferably be realized by summing motion-corrected nuclear medical image data sets for the different acquisition steps and/or by applying motion correction during reconstruction and/or already on the nuclear medical raw data sets.

In a preferred embodiment, attenuation correction (AC) based on an attenuation map may be applied to the at least one nuclear medical image data set, wherein the attenuation map is also motion-corrected according the motion data. Attenuation correction for nuclear medical imaging is well-known and can, preferably, also be applied here. In particular in cases where an attenuation map is only determined for one of the acquisition steps, the motion data may, of course, also be used to correct the attenuation map for the other acquisition steps, further improving image quality and spatial accuracy of correspondence.

As already mentioned, at least a part of the source data for each acquisition step may be provided by nuclear medical raw data and/or images derived therefrom. In embodiments, source data may additionally or alternatively be acquired by an acquisition device of an additional modality different from the nuclear medical imaging during the acquisition steps, wherein the acquisition device is registered to the imaging device. Hence, additional data, in particular data acquired anyways during the examination, can be used to provide robust, reliable and high-quality motion data and hence motion correction.

In especially preferred embodiments, the acquisition device may be an MRI device integrated into the imaging device, which may preferably be a PET device. That is, the imaging device may be a combined PET-MR device, as proposed in the state of the art. Here, two modalities, namely magnetic resonance imaging (MRI) and positron emission tomography (PET) are provided and, by integration, already registered to each other. Such combined imaging devices are often used to derive attenuation maps from the additional modality, here MRI, as, for example, described in an article by Daniel H. Paulus et al., "Whole-Body PET/MR Imaging: Quantitative Evaluation of a Novel Model-Based MR Attenuation Correction Method Including Bone", J Nucl Med. 56 (2015), pages 1061-1066. Such a combined device is also mainly discussed in US 8 781 195 B2. It is noted that also computer tomography (CT) may be used as additional modality, however, this is less preferred, since a high radiation dose would be applied to the patient when CT is excessively used.

However, also other additional modalities may be employed. For example, the acquisition device can also be a camera, in particular a 3D and/or terahertz camera, and/or a radar device and/or an ultrasound device. Such acquisition devices have already been proposed as a source of motion information.

In the context of MRI as additional modality, if attenuation correction is applied using the attenuation map, preferably, the attenuation map may be determined from attenuation correction MRI data acquired by the acquisition device during at least one of the acquisition steps, in particular at least the first acquisition step, wherein the attenuation correction MRI data is used as at least a part of the source data. In particular, acquisition correction MRI data may be acquired in each acquisition step to determine a respective attenuation map for the acquisition step. However, if acquisition correction MRI data are only acquired in one acquisition step, for the other acquisition steps, further, in particular diagnostic, MRI data may be acquired and at least partly used as source data. In both cases, MRI data acquired anyways in the workflow can additionally be used as source data for determining the motion data, allowing to avoid additional scans and/or acquisition devices for the source data. As already discussed in the art, for example in the article by Daniel H. Paulus cited above, dedicated attenuation correction sequences, in particular for Dixon techniques, may be used to acquire the attenuation correction MRI data, which may also be called AC MRI data. However, further MRI data acquired using other imaging sequences may alternatively or additionally be used as source data. It is noted that, if, during an acquisition step, no AC MRI data or further MRI data is anyways acquired, additional MRI data acquisition may, of course, be provided.

In preferred embodiments, source data from different modalities may be used to determine the motion data, in particular from modalities showing different anatomical features. For example, PET data and MRI data may both be used as source data. For example, if lesions visible in PET are not visible in MRI and/or anatomical structures visible in MRI are not visible in PET, by using source data from both modalities, motion information from different anatomical features can be combined to improve the quality of the motion data.

It is noted that motion data may also be determined time-resolved within the acquisition steps and used for motion correction of data acquired within the acquisition steps, as in principle known from the state of the art, to further improve image quality and evaluability.

The invention further concerns an imaging device for determining at least one nuclear medical image data set in nuclear medical imaging according to claim 8.

All comments and remarks regarding the computer-implemented method according to the invention analogously apply to the imaging device according to the invention, such that the same advantages can be achieved. In particular, the control device of the imaging device is configured to perform a method according to the invention. The imaging device may include acquisition components controlled by the acquisition unit to acquire data. The control device may, in particular, comprise at least one processor and/or at least one storage device. Further functional units may be provided to realize preferred embodiments, in particular those described in the dependent claims.

The imaging device is a combined imaging device (often also called hybrid imaging device), a PET-MR device.

A computer program according to the invention can be directly loaded into the storage device of a control device of an imaging device and enables the control device to perform the steps of a method according to the invention when the computer program is executed on the control device. The computer program may be stored on an electronically readable storage medium according to the invention, which thus comprises control information comprising a computer program according to the invention, such that, when the electronically readable storage medium is used in a control device of an imaging device, the control device is configured to perform the steps of a method according to the invention. The electronically readable storage medium may preferably be a non-transitory medium, for example a CD-ROM.

Other objects and features of the present invention will become apparent from the following detailed description considered in conjunction with the accompanying drawings. The drawings, however, are only principle sketches designed solely for the purpose of illustration and do not limit the invention. The drawings show:
- Fig. 1: a general flowchart of methods according to the invention,
- Fig. 2: a first acquisition schedule,
- Fig. 3: a second acquisition schedule,
- Fig. 4: a flowchart regarding an embodiment for determining motion data, and
- Fig. 5: an imaging device according to the invention.

In the following, embodiments of the current invention are discussed, wherein the nuclear medical imaging modality is positron emission tomography (PET) and a combined (or hybrid) imaging device is used, wherein the additional modality is magnetic resonance imaging (MRI). However, the invention is also applicable to other modalities and modality combinations, for example single photon emission computer tomography (SPECT). Other additional modalities like cameras or radar sensors may also be employed to provide source data for motion correction. It is, in particular, noted that the invention can also be applied to single nuclear medical imaging modalities, in particular PET, where the single nuclear imaging modality itself provides source data. Using a PET-MR device the imaging device is, however, preferred.

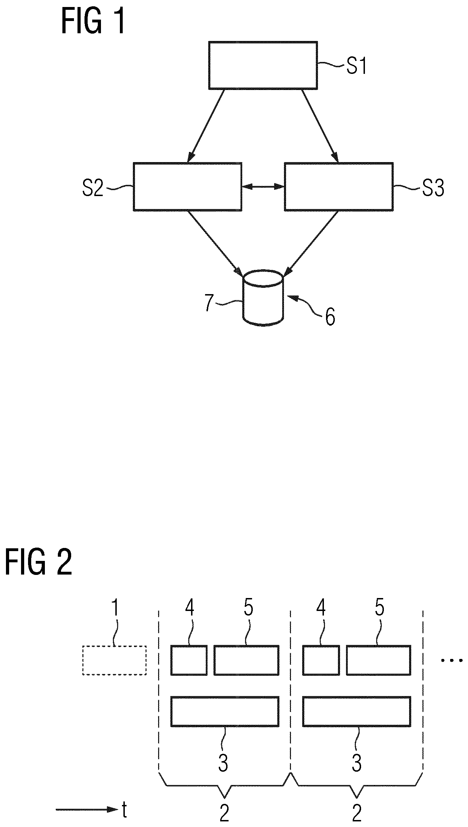

Fig. 1 shows a general flowchart for embodiments of the method according to the invention. Here, in a step S1, data are acquired by the imaging device. In the embodiments discussed here, a region of interest of a patient is to be examined, which is larger than the field of view of the nuclear medical imaging device, here PET device, of the imaging device. Hence, multiple table positions of the patient table of the imaging device (often also called bed positions) are predefined such that all subregions of the region of interest can be imaged. In particular, the examination to be performed may be a whole-body examination. For example, two to seven predefined patient table positions, which correspond to field of view positions with respect to the patient, may be used.

Nuclear medical raw data, in this case PET raw data, of the region of interest are not acquired in one pass or sweep of the region of interest using the predefined patient table positions, but in multiple acquisition steps, in particular multiple full sweeps of the region of interest, which may also be called multiple passes of the region of interest. For each of the acquisition steps, a nuclear medical raw data set, in particular a list-mode data set, is acquired. Here, in particular since multiple predefined positions of the field of view, in this case patient table positions, are successively used, there is always a time interval between the acquisition of nuclear medical raw data from the same sub-region. Of course, there may also be a pause between PET acquisitions of the different steps. For example, five predefined table positions may be used, where nuclear medical raw data are acquired for one minute in each acquisition step (pass). Hence, it will take at least four minutes until nuclear medical raw data will be acquired from the same subregion of the region of interest again. It should be noted at this point that the principles of the invention, as discussed here, can of course also be applied to cases where the patient table is continuously moved to sweep the region of interest.

Since the nuclear medical raw data sets of different acquisition steps are acquired at different points in time in step S1, motion effects, in particular regarding patient motion, may become relevant.

In preferred embodiments, the acquisition schedule is chosen such that dynamic nuclear medical imaging can be performed. In particular, each nuclear medical raw data set may be acquired in the same manner, that is, measurement is performed for the same acquisition times for each patient table position. In this manner, as will be further discussed below, nuclear medical image data sets may be determined for each acquisition step, providing a series of nuclear medical images describing the progression of the tracer, which was given to the patient long before the acquisition in step S1, in the region of interest.

An acquisition schedule is shown exemplarily in fig. 2. Here, the upper blocks relate to MRI acquisitions, while the lower blocks relate to PET acquisitions. First, as illustrated by block 1, MR localizer data are acquired, such that the combined PET-MRI acquisition can be planned. After planning, in a first acquisition step 2, PET raw data of a first PET raw data set (as nuclear medical data set) are acquired for all predefined patient table positions. In parallel, since the combined imaging device allows synchronous acquisition, MRI data are acquired, namely , according to block 4, attenuation correction MRI data and, according to block 5, further MRI data, in this case diagnostic MRI data.

In the example of fig. 2, this acquisition step 2 is repeated for a predefined number of times, for example three to six times, to, in particular, acquire, for each acquisition step 2, a PET raw data set using the same acquisition parameters.

It is noted that, in some cases, attenuation correction MRI data (AC MRI data) may only be acquired in one of the acquisition steps 2, however, acquisition in each acquisition step 2 is preferred. Furthermore, of course, the acquisition time for MRI data may, in this first example, also be shorter than the acquisition time for the PET data. Generally, attenuation correction MRI data is used to determine attenuation correction maps to apply attenuation correction to nuclear medical imaging raw and/or image data, in particular during reconstruction.

However, the invention can also be applied to other acquisition schedules, in which multiple PET raw data sets, optionally using different acquisition parameters, are acquired in different acquisition steps 2.

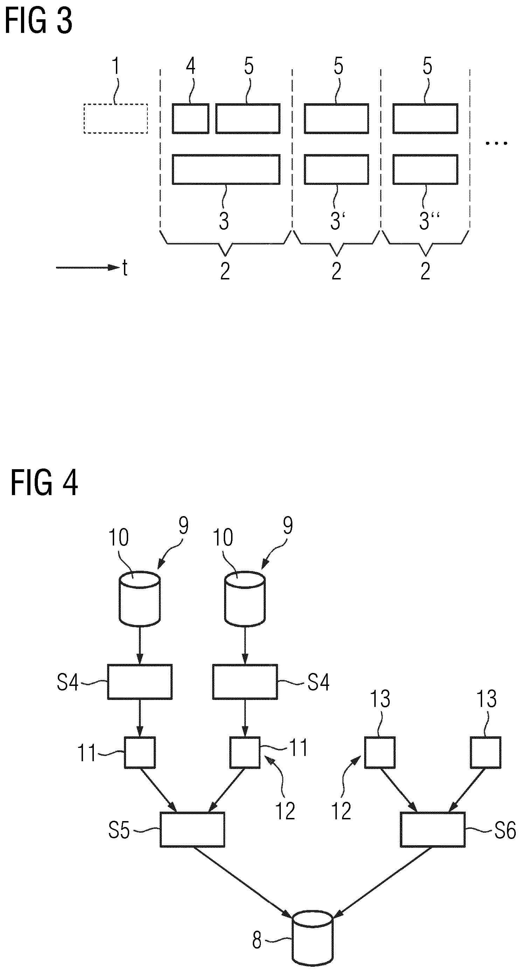

Fig. 3 shows an example where, again after the acquisition of localizer MRI data in block 1 and respective planning, in the first acquisition step 2, attenuation correction MRI data are acquired in block 4 and diagnostic MRI data are acquired in block 5. PET raw data of a full sweep of the region of interest are acquired according to block 3. However, in later acquisition steps 2, only further diagnostic MRI data are acquired in blocks 5, while in blocks 3' and 3", which may use shorter total acquisition time as blocks 5, further PET raw data sets for the following acquisition steps 2 are acquired, wherein the acquisition times for the different patient table positions may not match those for the first PET raw data set of the first acquisition step 2 or patient table positions may even be skipped. For example, blocks 3', 3" may serve to increase recorded PET data, as, for example, described in US 8 781 195 B2 already mentioned above. Also in such a case, the problem of patient motion occurring between the acquisition steps 2 exists when a full-count or at least multiple-count nuclear medical image data set, in this case PET data set, is to be determined.

Generally, in addition to the reconstruction of at least one nuclear medical image data set 6, in this case PET image data set 7, in a step S3, motion data describing the motion between acquisition steps 2 are determined from source data and used for motion correction in step S3. It is noted that motion data can also be used to adapt attenuation maps, if, like the case of fig. 3, attenuation correction MRI data are only acquired during one attenuation step. As source data, PET data and/or additional modality data, here MRI data, may be used, wherein preferably both PET data and MRI data may be used to determine the motion data, since both modalities show different anatomical features.

An exemplary flowchart for the determination of motion data 8 is shown in fig. 4 for a pair of acquisition steps 2. Here, the nuclear medical raw data sets 9, in this case PET raw data sets 10, for each of the acquisition steps 2 are used in steps S4 to determine preliminary reconstructed images 11, in this case preliminary backprojected images, as source data 12. These preliminary reconstructed images 11 may be attenuation corrected (AC) or not attenuation corrected (NAC) using attenuation maps derived from the attenuation correction MRI data already discussed above. For example, Dixon techniques may be used to acquire attenuation correction MRI data in blocks 4, as principally known in the state of the art.

In a step S5, the preliminary reconstructed images 11 are registered using a registration algorithm to determine first motion data.

On the other hand, MRI data 13, preferably attenuation correction MRI data, are used as source data 12, in particular as input to a step S6, where the corresponding MRI images are registered to determine second motion data. The first and second motion data are then statistically combined to yield the final motion data 8.

It is noted that the use of attenuation correction MRI data is preferred, since, usually, Dixon techniques are employed yielding material distributions, which can easily and robustly be registered in a registration algorithm in step S6. However, it is also possible to use further MRI data, in particular diagnostic MRI data, or even to register MRI images acquired using different magnetic resonance sequences and/or protocols in step S6, for example if, like the case in fig. 3, attenuation correction MRI data are not available for each acquisition step 2.

Motion data 8 can, of course, also be determined using other methods, for example optical flow algorithms and the like. Furthermore, further additional modalities may be used as a source of further motion data to be combined, for example cameras, in particular 3D cameras and/or terahertz cameras.

Returning to fig. 1, in step S3, the motion data 8 are used to perform motion correction regarding the different acquisition steps 2. Here, multiple approaches are conceivable within the current invention, wherein, for example, in less preferred embodiments, a correction may be applied to already reconstructed nuclear medical image data sets 6. In preferred embodiments, however, motion correction is applied before or during reconstruction. If, for example, iterative reconstruction algorithms are used in step S2, for example motion sensitive OSEM reconstruction, the intermediate image may be warped before a forward projection and the correction terms resulting from comparison of the nuclear medical raw data and the forward projected data may be unwarped after backward projection. In this case, motion correction may be applied in sinogram space. However, in preferred embodiments, the motion data may also be applied in listmode space by reordering the lines of response of the events according to the motion information.

In the preferred case of dynamic nuclear medical imaging, a series of nuclear medical image data sets 6 for all the acquisition steps 2 is preferably determined, wherein the motion correction leads to pixel-wise correspondence between the nuclear medical image data sets 6. Furthermore, also in the case of dynamic nuclear medical imaging, a full-count nuclear medical image data set 6 may be determined by summing up the motion-corrected nuclear medical image data sets for all the acquisition steps 2. In cases like illustrated in fig. 3, however, exactly one nuclear medical image data set 6, namely a full-count nuclear medical image data set 6, may be determined as the final result. In any case, the at least one nuclear medical image data set 6 is then provided for further processing, for example displaying on a display device of the imaging device, storing in a picture archiving system and/or further evaluation.

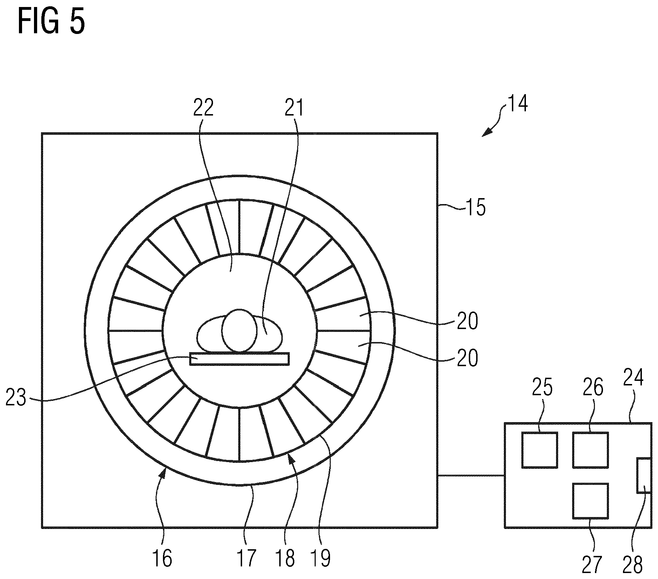

Fig. 5 shows an embodiment of an imaging device 14 according to the invention, in this case a PET-MR device 15 providing both modalities. In the schematical drawing of fig. 5, the acquisition device 16 for the additional modality, in this case the MRI device 17, is only schematically indicated and provides, as known, a central bore, where the nuclear medical imaging device 18, in this case PET device 19, is located coaxially to the MRI device 17. The PET device 19 comprises multiple PET detection units 20 facing each other and arranged in pairs about the longitudinal direction (perpendicular to the image plane of fig. 5). For example, the PET detection units 20 may comprise LSO crystals upstream of a photodiode array and/or an electrical amplifying circuit. Other concrete embodiments are also conceivable. An anatomical region of a patient 21 may be introduced into the field of view in the bore 22 of the imaging device 14 using a patient table 23, as already discussed above.

The operation of the imaging device 14 is controlled by a control device 24, whose functional structure is also partly indicated in fig. 5. Generally said, the control device 24 is configured to perform a method according to the invention.

The control device 24 comprises an acquisition unit 25 to control the MRI device 17 and the PET device 19 to acquire respective data. Of course, separate acquisition units 25 for both modalities may also be provided. Hence, the at least one acquisition unit 25 is configured to perform step S1 of fig. 1.

The control device 24 further comprises a reconstruction unit 26 for performing the reconstruction of step S2 and a motion correction unit 27 for performing the determination of motion data 8 and the motion correction according to step S3. Via an interface 28, the resulting at least one nuclear medical image data set 6 (PET image data set 7) may be provided, for example to a display device of the hybrid imaging device 14 (not shown).

Although the present invention has been described in detail with reference to the preferred embodiment, the present invention is not limited by the disclosed examples from which the skilled person is able to derive other variations without departing from the scope of the invention.

## Claims

1. Computer-implemented method for determining at least one nuclear medical image data set (6) in nuclear medical imaging using an imaging device (14), wherein the imaging device (14) is a combined PET-MR device,
- multiple nuclear medical raw data sets (9) of a region of interest of a patient (21) are acquired in respective acquisition steps (2) during the progression of a tracer in the region of interest,
- at least one nuclear medical image data set (6) is reconstructed from the nuclear medical raw data sets (9) for each acquisition step (2),
- the source data (12) is at least partly acquired by an MRI device (17) during the acquisition steps (2), the MRI device (17) being registered to the imaging device (14), wherein the source data (12) are acquired in parallel to the nuclear medical raw data sets (9),
- wherein the region of interest is larger than the field of view of the imaging device (14),
- wherein multiple sub-regions of the region of interest are defined by patient table positions of a patient table (23) on which the patient (21) is placed for examination in the imaging device (14),
- wherein in each of the acquisition steps (2) nuclear medical raw data of one sub-region of the multiple sub-regions are acquired,
- wherein the acquisition of nuclear medical raw data of a certain sub-region of the multiple sub-regions is performed at least two times in at least two acquisition steps (2), wherein a time interval passes between the at least two acquisition steps (2),
- wherein motion data (8) describing the motion of the patient (21) between the at least two acquisition steps (2) is determined from source data (12) describing the motion in the region of interest and motion correction is applied to the series of nuclear medical raw data sets (9) and/or the at least one nuclear medical image data set (6) according to the motion data (8).

2. Method according to claim 1, **characterized in that** the source data (12) relating to one of the acquisition steps (2) is registered to source data (12) from another acquisition step (2) to determine motion data (8) between the acquisition steps (2) and/or optical flow algorithms are used to determine the motion data (8).

3. Method according to one of the preceding claims, **characterized in that** attenuation correction based on an attenuation map is applied to the at least one nuclear medical image data set (6), wherein the attenuation map is also motion-corrected according to the motion data (8).

4. Method according to any of the preceding claims, **characterized in that**, if attenuation correction is applied using the attenuation map, the attenuation map is determined from attenuation correction MRI data acquired by the acquisition device (16) during at least one of the acquisition steps (2), in particular at least the first acquisition step (2), wherein the attenuation correction MRI data is used as at least a part of the source data (12).

5. Method according to claim 4, **characterized in that** acquisition correction MRI data are acquired in each acquisition step (2) to determine a respective attenuation map for the acquisition step (2), or that acquisition correction MRI data are only acquired in one acquisition step (2), wherein, for the other acquisition steps (2), further, in particular diagnostic, MRI data are acquired and at least partly used as source data (12).

6. Method according to one of the preceding claims, **characterized in that** source data (12) from different modalities is used to determine the motion data (8), in particular modalities showing different anatomical features.

7. Method according to one of the preceding claims, **characterized in that** the motion data (8) is also determined time-resolved within the acquisition steps (2) and used for motion correction of data acquired within the acquisition step (2).

8. Imaging device (14) for determining at least one nuclear medical image data set (6) in nuclear medical imaging, wherein the imaging device (14) is a combined PET-MR device, comprising a control device (24) having an acquisition unit (25), a reconstruction unit (26), a motion correction unit (27), wherein the imaging device (14) is configured to carry out a method according to any of the preceding claims.

9. Computer program, which performs the steps of a method according to claims 1 to 7 when the computer program is executed on a control device (24) of an imaging device (14).

10. Electronically readable storage medium, on which a computer program according to claim 9 is stored.

## Patentansprüche

1. Computerimplementiertes Verfahren zur Bestimmung mindestens eines nuklearmedizinischen Bilddatensatzes (6) bei der nuklearmedizinischen Bildgebung unter Verwendung einer Bildgebungsvorrichtung (14), wobei
- die bildgebende Vorrichtung (14)
eine kombinierte PET-MR-Vorrichtung ist,
- mehrere nuklearmedizinische Rohdatensätze (9) einer Interessensregion eines Patienten (21) während der Progression eines Tracers in der Interessensregion in den jeweiligen Erfassungsschritten (2) erfasst werden;
- für jeden Erfassungsschritt (2) mindestens ein nuklearmedizinischer Bilddatensatz (6) aus den nuklearmedizinischen Rohdatensätzen (9) rekonstruiert wird;
- die Quelldaten (12) während der Erfassungsschritte (2) zumindest teilweise von einer MRT-Vorrichtung (17) erfasst werden, wobei die MRT-Vorrichtung (17) bei der Bildgebungsvorrichtung (14) registriert wird, wobei die Quelldaten (12) parallel zu den nuklearmedizinischen Rohdatensätzen (9) erfasst werden,
- wobei die Interessensregion größer als das Sichtfeld der Bildgebungsvorrichtung (14) ist,
- wobei mehrere Teilregionen der Interessensregion durch Patiententischpositionen eines Patiententisches (23) definiert sind, auf dem der Patient (21) zur Untersuchung in der Bildgebungsvorrichtung (14) platziert wird,
- wobei in jedem der Erfassungsschritte (2) nuklearmedizinische Rohdaten einer Teilregion der mehreren Teilregionen erfasst werden,
- wobei die Erfassung nuklearmedizinischer Rohdaten einer bestimmten Teilregion der mehreren Teilregionen mindestens zweimal in mindestens zwei Erfassungsschritten (2) durchgeführt wird, wobei ein Zeitintervall zwischen den mindestens zwei Erfassungsschritten (2) vergeht,
- wobei Bewegungsdaten (8), die die Bewegung des Patienten (21) zwischen den mindestens zwei Erfassungsschritten (2) beschreiben, aus Quelldaten (12) bestimmt werden, die die Bewegung in der Interessensregion beschreiben, und gemäß den Bewegungsdaten (8) eine Bewegungskorrektur auf die Reihe von nuklearmedizinischen Rohdatensätzen (9) und/oder den mindestens einen nuklearmedizinischen Bilddatensatz (6) angewendet wird.

2. Verfahren nach Anspruch 1, **dadurch gekennzeichnet, dass** die Quelldaten (12) in Bezug auf einen der Erfassungsschritte (2) mit Quelldaten (12) aus einem anderen Erfassungsschritt (2) registriert werden, um Bewegungsdaten (8) zwischen den Erfassungsschritten (2) zu bestimmen, und/oder Algorithmen für optischen Fluss verwendet werden, um die Bewegungsdaten (8) zu bestimmen.

3. Verfahren nach einem der vorhergehenden Ansprüche, **dadurch gekennzeichnet, dass** eine Dämpfungskorrektur basierend auf einer Dämpfungskarte auf den mindestens einen nuklearmedizinischen Bilddatensatz (6) angewendet wird, wobei die Dämpfungskarte außerdem gemäß den Bewegungsdaten (8) bewegungskorrigiert wird.

4. Verfahren nach einem der vorhergehenden Ansprüche, **dadurch gekennzeichnet, dass**, falls eine Dämpfungskorrektur unter Verwendung der Dämpfungskarte angewendet wird, die Dämpfungskarte aus Dämpfungskorrektur-MRT-Daten bestimmt wird, die von der Erfassungsvorrichtung (16) während mindestens eines der Erfassungsschritte (2), insbesondere mindestens des ersten Erfassungsschritts (2), erfasst werden, wobei die Dämpfungskorrektur-MRT-Daten als mindestens ein Teil der Quelldaten (12) verwendet werden.

5. Verfahren nach Anspruch 4, **dadurch gekennzeichnet, dass** in jedem Erfassungsschritt (2) Erfassungskorrektur-MRT-Daten erfasst werden, um eine jeweilige Dämpfungskarte für den Erfassungsschritt (2) zu bestimmen, oder dass Erfassungskorrektur-MRT-Daten nur in einem Erfassungsschritt (2) erfasst werden, wobei für die anderen Erfassungsschritte (2) weitere, insbesondere diagnostische, MRT-Daten erfasst und zumindest teilweise als Quelldaten (12) verwendet werden.

6. Verfahren nach einem der vorhergehenden Ansprüche, **dadurch gekennzeichnet, dass** Quelldaten (12) aus verschiedenen Modalitäten verwendet werden, um die Bewegungsdaten (8) zu bestimmen, insbesondere Modalitäten, die unterschiedliche anatomische Merkmale zeigen.

7. Verfahren nach einem der vorhergehenden Ansprüche, **dadurch gekennzeichnet, dass** die Bewegungsdaten (8) außerdem innerhalb der Erfassungsschritte (2) zeitaufgelöst bestimmt und zur Bewegungskorrektur von innerhalb des Erfassungsschritts (2) erfassten Daten verwendet werden.

8. Bildgebungsvorrichtung (14) zum Bestimmen mindestens eines nuklearmedizinischen Bilddatensatzes (6) bei der nuklearmedizinischen Bildgebung, wobei die Bildgebungsvorrichtung (14) eine kombinierte PET-MR-Vorrichtung ist, die eine Steuervorrichtung (24) mit einer Erfassungseinheit (25), einer Rekonstruktionseinheit (26) und einer Bewegungskorrektureinheit (27) umfasst, wobei die Bildgebungsvorrichtung (14) dazu ausgelegt ist, ein Verfahren nach einem der vorhergehenden Ansprüche durchzuführen.

9. Computerprogramm, das die Schritte eines Verfahrens nach einem der Ansprüche 1 bis 7 ausführt, wenn das Computerprogramm auf einer Steuervorrichtung (24) einer Bildgebungsvorrichtung (14) ausgeführt wird.

10. Elektronisch lesbares Speichermedium, auf dem ein Computerprogramm nach Anspruch 9 gespeichert ist.

## Revendications

1. Procédé mis en œuvre par ordinateur de détermination d'au moins un ensemble (6) de données d'images médicales nucléaires dans une imagerie médicale nucléaire utilisant un dispositif (14) d'imagerie, dans lequel
- le dispositif (14) d'imagerie est un dispositif combiné PET-RM,
- de multiples ensembles (9) de données brutes médicales nucléaires d'une région à laquelle on s'intéresse d'un patient (21) sont acquises dans des stades (2) d'acquisition respectifs pendant la progression d'un traceur dans la région à laquelle on s'intéresse,
- au moins un ensemble (6) de données d'images médicales nucléaires reconstruit à partir des ensembles (9) de données brutes d'images nucléaires pour chaque stade (2) d'acquisition,
- la donnée (12) de source est acquise au moins en partie par un dispositif (17) d'IRM pendant les stades (2) d'acquisition, le dispositif (17) d'IRM étant référencé au dispositif (14) d'imagerie, dans lequel les données (12) de source sont acquises en parallèle avec les ensembles (9) de données brutes médicales nucléaires,
- dans lequel la région à laquelle on s'intéresse est plus grande que le champ de vue du dispositif (14) d'imagerie,
- dans lequel de multiples sous-régions de la région à laquelle on s'intéresse sont définies par des positions d'une table (23) de patient, sur laquelle le patient (21) est placé pour examen dans le dispositif (14) d'imagerie,
- dans lequel, dans chacun des stades (2) d'acquisition, des données brutes médicales nucléaires d'une sous-région des multiples sous-régions sont acquises,
- dans lequel l'acquisition de données brutes médicales nucléaires d'une certaine sous-région des multiples sous-régions est effectuée au moins deux fois dans au moins deux stades (2) d'acquisition, dans lequel un intervalle de temps se passe entre les au moins deux stades (2) d'acquisition,
- dans lequel une donnée (8) de mouvement, décrivant le mouvement du patient (21) pendant les au moins les deux stades (2) d'acquisition, est déterminée à partir de la donnée (12) de source décrivant le mouvement dans la région à laquelle on s'intéresse et une correction de mouvement est appliquée à la série d'ensembles (9) de données brutes médicales nucléaires et/ou à le au moins un ensemble (6) de données d'image médicale nucléaire en fonction de la donnée (8) de mouvement.

2. Procédé suivant la revendication 1, **caractérisé en ce que** la donnée (12) de source se rapportant à l'un des stades (2) d'acquisition est référencée à une donnée (12) de source d'un autre stade (2) d'acquisition pour déterminer une donnée (8) de mouvement entre les stades (2) d'acquisition et/ou des algorithmes optiques d'écoulement sont utilisés pour déterminer la donnée (8) de mouvement.

3. Procédé suivant l'une des revendications précédentes, **caractérisé en ce qu'**une correction d'atténuation sur la base d'une carte d'atténuation est appliquée au au moins un ensemble (6) de données d'image médicale nucléaire, dans lequel la carte d'atténuation est aussi à correction de mouvement en fonction de la donnée (8) de mouvement.

4. Procédé suivant l'une quelconque des revendications précédentes, **caractérisé en ce que**, si une correction d'atténuation est appliquée en utilisant la carte d'atténuation, la carte d'atténuation est déterminée à partir d'une donnée d'IRM de correction d'atténuation acquise par le dispositif (16) d'acquisition pendant au moins l'un des stades (2) d'acquisition, en particulier pendant au moins le premier stade (2) d'acquisition, la donnée d'IRM de correction d'atténuation étant utilisée à au moins une partie de la donnée (12) de source.

5. Procédé suivant la revendication 4, **caractérisé en ce que** les données d'IRM de correction d'acquisition sont acquises dans chaque stade (2) d'acquisition pour déterminer une carte d'atténuation respective pour le stade (2) d'acquisition, ou **en ce que** des données d'IRM de correction d'acquisition sont acquises seulement dans un stade (2) d'acquisition, dans lequel, pour les autres stades (2) d'acquisition en outre des données d'IRM, en particulier de diagnostic, sont acquises et utilisées au moins en partie comme donnée (12) de source.

6. Procédé suivant l'une des revendications précédentes, **caractérisé en ce qu'**une donnée (12) de source de modalités différentes est utilisée pour déterminer la donnée (8) de mouvement, en particulier de modalités montrant des caractéristiques anatomiques différentes.

7. Procédé suivant l'une des revendications précédentes, **caractérisé en ce que** la donnée (8) de mouvement est déterminée également d'une manière résolue dans le temps dans les stades (2) d'acquisition et utilisée pour une correction de mouvement de données acquises dans le stade (2) d'acquisition.

8. Dispositif (14) d'imagerie pour déterminer au moins un ensemble (6) de données d'images médicales nucléaires dans une imagerie médicale nucléaire, dans lequel le dispositif (14) d'imagerie est un dispositif combiné PET-RM comprenant un dispositif (24) de commande ayant une unité (25) d'acquisition, une unité (26) de reconstruction, une unité (27) de correction de mouvement, dans lequel le dispositif (14) d'imagerie est configuré pour effectuer un procédé suivant l'une quelconque des revendications précédentes.

9. Programme d'ordinateur, qui effectue les stades d'un procédé suivant l'une des revendications 1 à 7, lorsque le programme d'ordinateur est exécuté sur un dispositif (24) de commande d'un dispositif (14) d'imagerie.

10. Support de mémoire, déchiffrable électroniquement, sur lequel un programme d'ordinateur suivant la revendication 9 est mis en mémoire.
